# EUROPEAN PATENT APPLICATION

(11) **EP 4 335 467 A1**
(43) Date of publication of application: **13.03.2024**
(21) Application number: 22194849.0
(22) Date of filing: 09.09.2022
(51) Int. Cl.: A61L 27/20, A61L 27/50, A61L 27/52

(54) **A MEDICAL GRADE NANOFIBRILLAR CELLULOSE HYDROGEL FOR USE AS IMPLANTABLE MATERIAL**

(71) Applicant: UPM-Kymmene Corporation, 00100 Helsinki (FI)
(72) Inventor: Kuncova-Kallio, Johana, 00100 Helsinki (FI); Kiuru, Tony, 00100 Helsinki (FI); Paasonen, Lauri, 00100 Helsinki (FI); Luukko, Kari, 00100 Helsinki (FI); Kosonen, Mika, 00100 Helsinki (FI); Siltanen, Henri, 00100 Helsinki (FI)
(74) Representative: Berggren Oy

(57) **Abstract**

The present disclosure provides a medical grade nanofibrillar cellulose hydrogel comprising plant-based chemically and enzymatically unmodified nanofibrillar cellulose hydrogel having a concentration of the nanofibrillar cellulose in the range of 1.4-3.4% by weight, the nanofibrillar cellulose having a number-average diameter of fibrils and/or fibril bundles of 100 nm or less, and a storage modulus in the range of 1-35 Pa. The present disclosure also provides the medical grade nanofibrillar cellulose hydrogel for use as implantable material in a body, and a method for manufacturing medical grade nanofibrillar cellulose hydrogel.

## Description

### Field of the application

The present application relates to a medical grade nanofibrillar cellulose hydrogel, the medical grade nanofibrillar cellulose hydrogel for use in a body, and to a method for manufacturing the medical grade nanofibrillar cellulose hydrogel.

### Background

Medical implants are devices or tissues that are placed inside or on the surface of the body. Many implants are prosthetics, intended to replace missing body parts. Other implants deliver medication, monitor body functions, or provide support to organs and tissues. Implants comprise or are made of implantable material. Some implants are made from skin, bone or other body tissues. Others are made from metal, plastic, ceramic or other materials. Implants can be placed permanently or they can be removed once they are no longer needed.

The risks of medical implants include surgical risks during placement or removal, infection, and implant failure. Some people also have reactions to the materials used in implants

Similarly to medical implants, also dermal fillers are placed inside body or tissue and may be considered as implantable material. Dermal fillers offer anti-ageing and skin remodelling treatments previously only achievable with surgery. Dermal fillers are used in the form of a hydrogel that can be injected into the skin. They are substantially cheaper, safer and more painless than surgical cosmetic procedures. There are various types of dermal fillers available on the market today. Upon injection, these fillers act as filling materials to provide volume to the skin, thereby providing the desired aesthetic effect of improving the skin's contour and reducing depressions in the skin due to scars, injury or lines

One example of a material used as a filler in a body is hyaluronic acid. However, upon administration to an individual, hyaluronic acid undergoes enzymatic degradation by various enzymes, such as hyaluronidase, glucoronidase, and glucosidase, or non-enzymatic degradation, and thus does not maintain its original viscosity or desired residence time *in vivo.*

Some fillers are diluted with lidocaine or saline to add anaesthetic properties to the dermal filler and/or change its flow and viscoelastic properties. Some dermal fillers suffer from the drawback that they are not stable over time.

Another drawback often caused by implantable materials is foreign substance reaction, which causes inflammation. Also degradation of implantable material, such as chemical, enzymatical and/or mechanical degradation, leads to frequent re-administration. Implantable materials may change their physical and chemical properties, such as by shrinking, over time when present in a body.

There is a need for implantable materials, which are tolerated by a body and which maintain their composition and form in the body.

There is a need for implant and dermal filler compositions which provide an optimal balance of volumizing capacity, longevity and ease of injection. Alternative, additional, and/or improved dermal fillers, uses thereof, and methods for the production thereof, are desirable.

There is also a need for high quality products and manufacturing methods thereof, which can fulfil the medical device regulations and which can be used in various medical applications including inserting the material into a body, especially human body.

### Summary

It was found out how to manufacture medical grade nanofibrillar cellulose hydrogels, which can be used as implantable material for inserting, such as injecting, safely into a body. The hydrogels exhibited very high purity in respect of microbial purity and biocompatibility. The nanofibrillar cellulose in the hydrogels is homogenous and it maintains its nanoscale structure when applied thus exhibiting suitable properties for demanding medical uses. The process and the obtained medical grade nanofibrillar cellulose overcome drawbacks of prior art.

There were however challenges for preparing such medical grade products in industrial scale, as the processes of preparing nanofibrillar cellulose often require large equipment and different process steps, which are generally carried out at different locations. It is not straightforward to implement such processes in required clean conditions.

The present disclosure provides a medical grade nanofibrillar cellulose hydrogel comprising plant-based chemically and enzymatically unmodified nanofibrillar cellulose hydrogel having a concentration of the nanofibrillar cellulose in the range of 1.4-3.4% by weight, the nanofibrillar cellulose having a number-average diameter of fibrils and/or fibril bundles of 100 nm or less, and a storage modulus in the range of 1-40 Pa determined by a rotational rheometer using plate geometry at a consistency of 0.5% by weight in aqueous medium at 22±1°C and/or a storage modulus in the range of 0.7-20 Pa determined by a rotational rheometer using vane geometry at a consistency of 0.5% by weight in aqueous medium at 22±1°C.

The present disclosure provides the medical grade nanofibrillar cellulose hydrogel for use as an implantable material in a body.

The present disclosure provides a method for manufacturing medical grade nanofibrillar cellulose hydrogel for use in a body, the method comprising
- providing a suspension of plant-based cellulose pulp,
- pre-refining the cellulose pulp suspension with a refiner to obtain a pre-refined cellulose having SR value 87 or more, such as SR value in the range of 87-95,
- dispersing the pre-refined cellulose pulp to obtain a concentration of pre-refined cellulose in the range of 1.6-3.6% by weight,
- disintegrating the pre-refined cellulose pulp by a disintegrating device at said concentration to obtain medical grade nanofibrillar cellulose hydrogel,
- recovering the medical grade nanofibrillar cellulose hydrogel from the disintegrating device without adjusting the concentration,
- to obtain nanofibrillar cellulose hydrogel having a concentration of nanofibrillar cellulose in the range of 1.4-3.4% by weight.

The present disclosure provides a medical grade nanofibrillar cellulose hydrogel obtained by the method.

The main embodiments are characterized in the independent claims. Various embodiments are disclosed in the dependent claims. The embodiments and examples disclosed herein are mutually freely combinable unless otherwise explicitly stated.

The present nanofibrillar cellulose hydrogels could be inserted into a body, more particularly to a target in a body, for example by injecting, and it was found out that in the body the hydrogels could support tissues by the effect of their stiffness. The target may be a specific tissue or location, such as a location in or near a tissue and/or a point which needs supporting and/or filling.

By the present method for manufacturing medical grade nanofibrillar cellulose hydrogel it was possible to obtain hydrogels which exhibited physiological properties of the target tissues or supporting tissues required in the target. The viscoelastic properties of the present hydrogels were found to be suitable for such use. The hydrogels maintained their form and remained in the body for a prolonged time, for at least a month, which makes them ideal for specific medical applications. Such applications include tissue-supporting applications, which may be medical, prosthetic and/or aesthetical.

The present nanofibrillar cellulose hydrogel is non-toxic non-animal and non-microbial based biocompatible material . In tests the hydrogel caused practically no foreign substance reactions or encapsulation, which is extremely rare for any insertable, injectable or implantable materials. The surface of the hydrogel was however reinforced in the body, which further supported the original form of the implanted hydrogel. The present nanofibrillar cellulose is also biodurable and cellulose resorption in a body is slow, as body cannot synthesize cellulases required to degrade cellulose. It was experimentally shown that the present hydrogels maintained their original shape in a body for prolonged time.

It was possible to obtain final products, which are ready for injection or other insertion. Furthermore, the self-life of the obtained products was very long. The medical grade products could be obtained by using the nanofibrillar cellulose as the only reinforcing material in the product, so no other polymeric materials were required, and at the simplest the medical products contained only water and nanofibrillar cellulose.

The present nanofibrillar cellulose hydrogels are ideal for inserting into a body by injecting, due to the shear-thinning properties of the hydrogel material. The shear-thinning property of the hydrogel enables injecting the hydrogels into a body into a shape of specific structures, such as elongated structures, which restore the high stiffness after the injection. The hydrogel can be later removed from the body by a simple surgery or even by suction, if desired, as it is not irreversibly bound to tissues.

### Brief description of the figures

- Figure 1A: shows gel nodules immediately of the injection (both side).
- Figure 1B: shows a gel nodule immediately of the injection.
- Figure 2A: shows macroscopical observation on the NFC injection sites 28 days after injection (#1A).
- Figure 2B: shows the NFC hydrogel after removing from subcutaneous tissue for weighing.

### Detailed description

In this specification, percentage values, unless specifically indicated otherwise, are based on weight (w/w, by weight, or wt%). If any numerical ranges are provided, the ranges include also the upper and lower values. The open term "comprise" also includes a closed term "consisting of" as one option. The diameters disclosed herein, unless specifically indicated otherwise, refer to the smallest diameter, and may be presented as average or number-average diameter and may be determined microscopically.

The present disclosure provides a medical grade nanofibrillar cellulose hydrogel comprising plant-based, such as wood-based, chemically and enzymatically unmodified nanofibrillar cellulose, which may be called as native nanofibrillar cellulose. The hydrogel may have a concentration of the nanofibrillar cellulose in the range of 1.4-3.4% by weight, such as 1.45-3.0% by weight. It was found out that the present native nanofibrillar cellulose at these concentrations was injectable into a body, for example with a syringe equipped with a needle, and the injectable/injected hydrogel could form a stable implant in the body. For example the stiffness of the material and well as stability were appropriate for the present uses.

Medical grade as used herein refers to material acceptable and suitable for use with living tissue, especially material which can be inserted, implanted and/or injected into a body or a tissue. Medical grade material is prepared at clean room conditions and the quality of the materials throughout the manufacturing process are monitored and controlled, such as for microbial quality, purity and other related properties. For example endotoxin content is controlled and maintained at low level. Medical grade material shall be safe, inert and biocompatible, which includes for example that it is non-toxic and it does not cause undesired reactions when in contact with living tissue, such as foreign substance reaction, allergic reaction, cytotoxic reaction, irritation or sensitization, or the like.

Due to the controlled preparation method the product is substantially free of impurities and contaminants, or the impurities and contaminants may be under detection level. The medical grade nanofibrillar cellulose hydrogel preferably comprises bacterial endotoxins 5.5 EU/g (ml) or less determined according to Ph.Eur. 2.6.14. Such material can be obtained with the process described herein.

The present medical grade nanofibrillar cellulose hydrogel may be provided for use in or on a body. The body may be animal body, such as human and/or non-human animal. Non-human animal may be for example domestic animals and farmed animals, or other animal. Especially for human body there are various applications, which may utilize the present hydrogels, such as medical applications for supporting tissues, which may be used for treating medical conditions, and aesthetical applications such as plastic surgery, and combinations thereof. The present medical grade nanofibrillar cellulose hydrogels are especially suitable for treating human subjects.

The present medical grade hydrogels may be used internally, i.e. inserted into a body, but they may be also used externally, such as applied topically, for example applied onto a wound or other damaged area, for example onto skin, or for other applicable medical uses.

### Nanofibrillar cellulose

Nanofibrillar cellulose (NFC) refers to isolated cellulose fibrils and/or fibril bundles derived from cellulose raw material, and it has a capability of forming viscous hydrogel in water. Nanofibrillar cellulose production techniques may be based on disintegrating fibrous raw material, such as grinding of aqueous dispersion of pulp fibers to obtain nanofibrillated cellulose. After the grinding or homogenization process, the obtained nanofibrillar cellulose material is a dilute viscoelastic hydrogel. The obtained material usually exists at a relatively low concentration homogeneously distributed in water due to the disintegration conditions. In an aqueous environment, a dispersion of cellulose nanofibrils forms a viscoelastic hydrogel network. The gel is formed already at relatively low concentrations of for example 0.05-0.2% (w/w) by dispersed and hydrated entangled fibrils. The viscoelasticity of the NFC hydrogel may be characterized for example with dynamic oscillatory rheological measurements.

Because of its nanoscale structure nanofibrillar cellulose has unique properties which enable functionalities which cannot be provided by conventional fibrous cellulose or for example synthetic fibers or fibrils. It is possible to prepare materials and products which exhibit different properties than conventional products or products using conventional cellulosic materials or other polymeric materials. However, because of the nanoscale structure nanofibrillar cellulose is also a challenging material. For example handling or adjusting the concentration of nanofibrillar cellulose may be difficult, and/or the properties of the nanofibrillar cellulose are prone to deteriorate in such processes.

In general nanofibrillar cellulose may be manufactured from various sources, which may be plant or microbial. Plant cellulose may comprise or consist of wood cellulose. Wood cellulose may be used for preparing a variety of different nanofibrillar products, and the properties of the final nanofibrillar cellulose can be affected by the choice of raw material, process conditions, used methods, modifications and other conditions and method steps. For example the present wood cellulose is specifically refined and fibrillated, which may be controlled to adjust the final properties of the cellulose. Wood cellulose is obtained from cellulose fibers originating from secondary cell walls, which cellulose is essentially crystalline with degree of crystallinity of at least 55%.

Wood may be from softwood tree such as spruce, pine, fir, larch, douglas-fir or hemlock, or from hardwood tree such as birch, aspen, poplar, alder, eucalyptus, oak, beech or acacia, or from a mixture of softwoods and hardwoods. The present nanofibrillar cellulose is preferably obtained from hardwood pulp. In one example the hardwood is birch. In one example the wood pulp is chemical pulp, which is obtained by a chemical pulping process. Chemical pulp is preferred for the products disclosed herein.

Nanofibrillar cellulose, including the cellulose fibrils and/or fibril bundles, is characterized by a high aspect ratio (length/diameter). The average length of nanofibrillar cellulose (the median length of particles such as fibrils and/or fibril bundles) may exceed 1 µm, and in most cases it is 50 µm or less. If the elementary fibrils are not completely separated from each other, the entangled fibrils, such as fibril bundles, may have an average total length for example in the range of 1-100 µm, 1-50 µm, or 1-20 µm. This applies especially for native grades of fibrils which are not shortened or digested, for example chemically, enzymatically or mechanically. However, if the nanofibrillar material is highly fibrillated, the elementary fibrils may be completely or almost completely separated and the average fibril length is shorter, such as in the range of 1-10 µm or 1-5 µm. Strongly derivatized nanofibrillar cellulose may have a shorter average fibril length, such as in the range of 0.3-50 µm, such as 0.3-20 µm, for example 0.5-10 µm or 1-10 µm. Especially shortened fibrils, such as enzymatically or chemically digested fibrils, or mechanically highly treated material, may have an average fibril length of less than 1 µm, such as 0.1-1 µm, 0.2-0.8 µm or 0.4-0.6 µm. The fibril length and/or diameter may be determined with several techniques, such as by microscopy. A suitable imaging software may be used. Fibril thickness and width distribution may be measured by image analysis of microscope images, such as images from a field emission scanning electron microscope (FE-SEM), a transmission electron microscope (TEM), such as a cryogenic transmission electron microscope (CRYO-TEM), or an atomic force microscope (AFM). In general AFM and TEM, especially CRYO-TEM, suit best for nanofibrillar cellulose grades with narrow fibril diameter distribution. From Cryo-TEM images, also the bundled structure can be seen.

If high aspect ratio is desired, it is not desired to shorten the fibril length so it may not be desired to use chemically and/or enzymatically digested cellulose, or such highly mechanically fibrillated material that the fibrils are already shortened. A low aspect ratio usually can be detected as decreased viscosity of an NFC dispersion.

The average lengths and/or diameters disclosed herein may be number-average lengths and/or diameters, in regard of the NFC but also in regards of other applicable entities, such as microbeads, particles, cells, cell-derived products, aggregates and the like discussed herein. However for spherical or substantially spherical entities or particles a volume median particle size or diameter may be used. These dimensions may be determined microscopically, as discussed herein.

A specific software may be used. The term "fibrils" also includes fibril bundles, where applicable. The mechanically disintegrated cellulosic material may contain a small amount of cellulose which is not fully separated into elemental fibrils but is still in the form of fibril bundles.

The nanofibrillar cellulose may be characterized by the average diameter (or width), or by the average diameter together with the viscosity, such as zero shear viscosity. The average diameter (width) of nanofibrillar cellulose in general is less than 1 µm, or 500 nm or less, such as in the range of 1-500 nm, but preferably 200 nm or less, even 100 nm or less or 50 nm or less, such as in the range of 1-200 nm, 2-200 nm, 2-100 nm, or 1-50 nm, even 1-20 for highly fibrillated material. The diameters disclosed herein refer to fibrils and/or fibril bundles. The smallest fibrils are in the scale of elementary fibrils, the average diameter being typically in the range of 4-12 nm. The dimensions and size distribution of the fibrils depend inter alia on chemical modification and the disintegration method and efficiency. In case of highly disintegrated native nanofibrillar cellulose, the average fibril and/or fibril bundle diameter may be in the range of 2-200 nm or 2-100 nm, for example in the range of 10-50 nm. Nanofibrillar cellulose is characterized by a large specific surface area and a strong ability to form hydrogen bonds. In water dispersion, the nanofibrillar cellulose typically appears as either light or turbid gel-like material. Depending on the fiber raw material, nanofibrillar cellulose obtained from plants, especially wood, may also contain small amounts of other plant components, especially wood components, such as hemicellulose or lignin. The amount is dependent on the plant source. Depending on the raw material source, such as hardwood pulp vs. softwood pulp, different polysaccharide composition exists in the final nanofibrillar cellulose product.

The native nanofibrillar cellulose suitable for the uses described herein has a number average fibril diameter in the range of 1-100 nm. In one example said nanofibrillar cellulose has a number average fibril diameter in the range of 1-50 nm, such as 2-20 nm or 5-30 nm.

In general cellulose nanomaterials may be divided into categories according to TAPPI W13021, which provides standard terms for cellulose nanomaterials. Not all of these materials are nanofibrillar cellulose. Two main categories are "Nano objects" and "Nano structured materials". Nanofibrillar materials include "Cellulose microcrystals" (sometimes called as CMC) having a diameter of 10-12 µm and length:diameter ratio (L/D) <2, and "Cellulose microfibrils" having a diameter of 10-100 nm and a length of 0.5-50 µm. Nano objects include "Cellulose nanofibers", which can be divided into "Cellulose nanocrystals" (CNC) having a diameter of 3-10 nm and L/D >5, and "Cellulose nanofibrils" (CNF or NFC), having a diameter of 5-30 nm and L/D >50.

Different types of nanofibrillar cellulose may be categorized based on three main properties: (i) size distribution, length and/or diameter (ii) chemical composition, and (iii) rheological properties. To fully describe a type, two or more properties may be used in parallel. Examples of different types include native (chemically and/or enzymatically unmodified) NFC, oxidized NFC (high viscosity), oxidized NFC (low viscosity), carboxymethylated NFC and cationized NFC.

One way to characterize the nanofibrillar cellulose is to use the viscosity of an aqueous solution or dispersion containing said nanofibrillar cellulose. The viscosity may be for example Brookfield viscosity or zero shear viscosity. The specific viscosity, especially zero shear viscosity, can be used to distinguish nanofibrillar cellulose from non-nanofibrillar cellulose, and/or to define the fibrillation degree. A high zero shear viscosity usually corresponds to a high fibrillation degree, to a high aspect ratio and/or to a low fibril diameter. Brookfield viscosity cannot properly characterize the shear-thinning properties, so it can be used for example for comparing different batches and in quality control.

The nanofibrillar cellulose hydrogels exhibit characteristic rheological properties, which can be evaluated for example by monitoring viscosity as a function of shear stress or shear rate. For example they are shear-thinning or pseudoplastic non-Newtonian materials, which may be considered as a special case of thixotropic behavior, which means that their viscosity depends on the speed or force by which the material is deformed. When measuring the viscosity in a rotational rheometer, the shear-thinning behavior is seen as a decrease in viscosity with increasing shear rate. At low enough shear rates, shear thinning fluids will show a constant viscosity value, η0, termed the zero shear viscosity or zero shear viscosity plateau. The zero-shear-rate viscosity is a limiting value that cannot be measured directly; rather, it must be estimated by extrapolation from several measurements at different shear rates. The zero shear viscosity value is determined from the constant region of the measured curve and it represents the situation wherein the shear rate approaches zero.

Rheometers that control the applied shear stress or shear strain are called rotational or shear rheometers, and can be used to measure the way in which a liquid, suspension or slurry flows in response to applied forces. They are used for shear-thinning fluids which cannot be defined by a single value of viscosity. The rheometer may use a rotor with a specific geometry, such as vane or plate geometry. The measurements carried out by vane and plate geometry provide different values, which may not be directly comparable. The measurements may be carried out in pure water at pH 7 at 25±1°C or 22±1°C, and at a standard consistency/concentration of the nanofibrillar cellulose of 0.5% by weight.

The nanofibrillar cellulose may have a zero-shear viscosity in the range of 1000-50000 Pa s, such as 1500-40000 Pa s, 1500-20000 Pa·s, 1500-10000 Pa s or 1500-6000 Pa·s, determined by a rotational rheometer using plate geometry at a consistency of 0.5% by weight in aqueous medium at 22±1°C. The nanofibrillar cellulose may have a zero-shear viscosity in the range of 370-3000 Pa·s, 370-2700 Pa·s, or 370-2100 Pa·s, such as 750-3000 Pa·s, 750-2700 Pa·s, or 750-2100 Pa·s, for example 1300-1800 Pa·s, determined by a rotational rheometer using vane geometry at a consistency of 0.5% by weight in aqueous medium at 22±1°C.

The hydrogels show plastic behavior, which means that a certain shear stress (force) is required before the material starts to flow readily. This critical shear stress is often called the yield stress (fracture strength). The yield stress can be determined from a steady state flow curve measured with a stress controlled rheometer. When the viscosity is plotted as function of applied shear stress, a dramatic decrease in viscosity is seen after exceeding the critical shear stress. The zero shear viscosity and the yield stress are the most important rheological parameters to describe the suspending power of the materials. These two parameters separate the different grades quite clearly and thus enable classification of the grades. A rotational rheometer, such as a stress controlled rotational rheometer, can be used to measure both zero shear viscosity and yield stress as well as other rheological properties.

The nanofibrillar cellulose may have a yield stress in the range of 0.5-10 Pa, such as 0.5-7.0 Pa, 0.7-7.0 Pa, or 0.7-3.5 determined by a rotational rheometer using plate geometry at a consistency of 0.5% by weight in aqueous medium at 22±1°C. The nanofibrillar cellulose may have a yield stress in the range of 0.2-3.5 Pa, 0.2-3.0 Pa or 0.2-2.0 Pa, such as 0.7-2.0 Pa or 0.2-1 Pa, determined by a rotational rheometer using vane geometry at a consistency of 0.5% by weight in aqueous medium at 22±1°C.

In one embodiment the nanofibrillar cellulose has a zero-shear viscosity in the range of 1500-50000 Pa·s and a yield stress in the range of 0.5-10 Pa, determined by a rotational rheometer using plate geometry at a consistency of 0.5% by weight in aqueous medium at 22±1°C. In one embodiment the nanofibrillar cellulose has a zero-shear viscosity in the range of 1500-40000 Pa·s and a yield stress in the range of 0.5-7.0 Pa, determined by a rotational rheometer using plate geometry at a consistency of 0.5% by weight in aqueous medium at 22±1°C.

In one embodiment the nanofibrillar cellulose has a zero-shear viscosity in the range of 370-3000 Pa·s and a yield stress in the range of 0.2-3.5 Pa, determined by a rotational rheometer using vane geometry at a consistency of 0.5% by weight in aqueous medium at 22±1°C. In one embodiment the nanofibrillar cellulose has a zero-shear viscosity in the range of 750-3000 Pa·s and a yield stress in the range of 0.7-3.5 Pa, determined by a rotational rheometer using vane geometry at a consistency of 0.5% by weight in aqueous medium at 22±1°C.

In one embodiment the nanofibrillar cellulose has a Brookfield viscosity in the range of 6000-12000 mPa·s measured with SCAN-P 50:84 method at 20°C±1°C at a consistency of 0.8% (w/w) and at 10 rpm. In one example the apparent viscosity of the nanofibrillar cellulose is measured with a Brookfield viscometer (Brookfield viscosity) or another corresponding apparatus. Suitably a vane spindle (number 73) is used. There are several commercial Brookfield viscometers available for measuring apparent viscosity, which all are based on the same principle. Suitably RVDV spring (Brookfield RVDV-III) is used in the apparatus. A sample of the nanofibrillar cellulose is diluted to a concentration of 0.8% by weight in water and mixed for 10 min. The diluted sample mass is added to a 250 ml beaker and the temperature is adjusted to 20°C±1°C, heated if necessary and mixed. A low rotational speed 10 rpm is used. In general Brookfield viscosity may be measured at 20°C±1 °C, at a consistency of 0.8% (w/w) and at 10 rpm.

The stiffness of the final hydrogel may be adjusted and/or provided according to the target, such as target tissue and/or desired effect in the target. The stiffness may be adjusted to be equivalent or substantially equivalent to the stiffness of a tissue in the target. The stiffness may be adjusted to be higher than the stiffness of a tissue in the target, especially when it is desired to support the target, a tissue and/or a body part. The storage modulus of human tissues may vary in the range of 1-10000000 Pa, such as 10-10000 Pa for most soft tissues. The storage modulus of the medical hydrogel at its final concentration may be adjusted to obtain the desired effect, for example to be equivalent or substantially equivalent to, or to be higher or lower than, the target tissue or tissues.

The stiffness of the nanofibrillar cellulose hydrogels can be evaluated from viscoelastic measurements of the gels. The storage modulus of the present hydrogels is in the range of 1-50 Pa, 1-40 Pa, or 1-35 Pa, such as in the range of 1-31 Pa, 3-50 Pa, 3-40 Pa, 3-35 Pa, 3-31 Pa, for example 3-15 Pa, 3-10 Pa, 3-8 Pa, 8-50 Pa, 8-40 Pa, 8-35 Pa, 8-31 Pa or 8-15 Pa determined by a rotational rheometer using plate geometry at a consistency of 0.5% by weight in aqueous medium at 22±1°C. Storage modulus of 8 Pa or more can be used when high stiffness is required, such as in supportive use.

The storage modulus may be in the range of 0.7-20 Pa determined by a rotational rheometer using vane geometry at a consistency of 0.5% by weight in aqueous medium at 22±1°C, such as in the range of 0.7-15.0 Pa, 0.7-12.0 Pa, 0.7-10.0 Pa, such as 0.7-7 Pa, for example 1.0-7 Pa, 2.0-7 Pa, or 2-5 Pa. In one embodiment the nanofibrillar cellulose has a storage modulus in the range of 1-20 Pa, such as 1-15 Pa, 1-12 Pa, 1-10 Pa, 2-20 Pa, 2-15 Pa, 2-12 Pa, or 2-10 Pa, determined by a rotational rheometer using vane geometry at a consistency of 0.5% by weight in aqueous medium at 22±1°C. Storage modulus of 2 Pa or more can be used when high stiffness is required, such as in supportive use. The storage modulus, as well as other rheological properties, is measured at standard conditions. At the concentration of the hydrogel the storage modulus is higher and at the level corresponding to the target conditions or the desired stiffness of the gel at the target.

Nanofibrillar cellulose may be characterized also with a loss modulus. In one embodiment the nanofibrillar cellulose has a loss modulus in the range of 0.5-4.0 Pa, such as 1.0-4.0 Pa or 1.0-2.0 Pa, determined by a rotational rheometer using plate geometry at a consistency of 0.5% by weight in aqueous medium at 22±1°C. Nanofibrillar cellulose may be characterized also with a loss modulus. In one embodiment the nanofibrillar cellulose has a loss modulus in the range of 0.15-1.20 Pa, such as 0.20-0.50 Pa, determined by a rotational rheometer using vane geometry at a consistency of 0.5% by weight in aqueous medium at 22±1°C.

The rheometers and methods disclosed herein may be used for determining zero-shear viscosity, yield stress/fracture strength, storage modulus, and loss modulus. The rheometer may provide a specific measuring program for measuring each of the properties.

In one example rheometer viscosity of the nanofibrillar cellulose dispersion is measured at 22°C with a stress controlled rotational rheometer (such as AR-G2, HR-10 or HR-1 by TA Instruments, UK), which may be equipped with a narrow gap vane geometry (diameter 28 mm, length 42 mm) in a cylindrical sample cup having a diameter of 30 mm. After loading the samples to the rheometer they are allowed to rest for 5 min before the measurement is started. The steady state viscosity is measured with a gradually increasing shear stress (proportional to applied torque) and the shear rate (proportional to angular velocity) is measured. The reported viscosity (=shear stress/shear rate) at a certain shear stress is recorded after reaching a constant shear rate or after a maximum time of 2 min. The measurement is stopped when a shear rate of 1000 s⁻¹ is exceeded.

In another example rheological measurements of the hydrogel samples are carried out with a stress controlled rotational rheometer (AR-G2, HR-10 or HR-1 by TA instruments, UK) equipped with 20 mm plate geometry. After loading the samples to the rheometer, 1 mm gap, they are allowed to settle for 5 min before the measurement was started. Storage modulus may be measured with gradually increasing shear stress in a range of 0.01-100 Pa at the frequency0.1 Hz, at 22°C.

The nanofibrillar cellulose may also be characterized by turbidity of the dispersion. Turbidity is the cloudiness or haziness of a fluid caused by individual particles (total suspended or dissolved solids) that are generally invisible to the naked eye. There are several practical ways of measuring turbidity, the most direct being some measure of attenuation (that is, reduction in strength) of light as it passes through a sample column of water. The alternatively used Jackson Candle method (units: Jackson Turbidity Unit or JTU) is essentially the inverse measure of the length of a column of water needed to completely obscure a candle flame viewed through it.

Turbidity may be measured quantitatively using optical turbidity measuring instruments. There are several commercial turbidometers available for measuring turbidity quantitatively. In the present case the method based on nephelometry may be used, and the measurement may be carried out at 20°C±1°C a consistency of 0.1% (w/w) in aqueous medium, such as pure water. The measurement may be carried out according to ISO 7027. The units of turbidity from a calibrated nephelometer are called Nephelometric Turbidity Units (NTU). The measuring apparatus (turbidometer) is calibrated and controlled with standard calibration samples, followed by measuring of the turbidity of the diluted NFC sample.

In one turbidity measurement method, a nanofibrillar cellulose sample is diluted in water, to a concentration below the gel point of said nanofibrillar cellulose, and turbidity of the diluted sample is measured. Said concentration where the turbidity of the nanofibrillar cellulose samples is measured is 0.1%. For example HACH P2100 Turbidometer with a 50 ml measuring vessel may be used for turbidity measurements. The dry matter of the nanofibrillar cellulose sample is determined and 0.5 g of the sample, calculated as dry matter, is loaded in the measuring vessel, which is filled with tap water to 500 g and vigorously mixed by shaking for about 30 s. Without delay the aqueous mixture is divided into 5 measuring vessels, which are inserted in the turbidometer. Three measurements on each vessel are carried out. The mean value and standard deviation are calculated from the obtained results, and the final result is given as NTU units.

One way to characterize nanofibrillar cellulose is to define both the viscosity and the turbidity. Low turbidity refers to small size of the fibrils, such as small diameter, as small fibrils scatter light poorly. In general as the fibrillation degree increases, the viscosity increases and at the same time the turbidity decreases. This happens, however, until a certain point. When the fibrillation is further continued, the fibrils finally begin to break and shorten, and therefore cannot form a strong network anymore. Therefore, after this point, both the turbidity and the viscosity begin to decrease.

The turbidity of the present medical grade nanofibrillar cellulose hydrogel may be in the range of 110-172 NTU, such as 130-170 NTU or 115-150 NTU, measured by nephelometry according to SFS-EN ISO 7027.

### Manufacture of the medical grade nanofibrillar cellulose hydrogel

The present disclosure provides a method for manufacturing medical grade nanofibrillar cellulose hydrogel.

The method comprises providing a suspension of plant-based cellulose pulp, preferably having a pH in the range of 6.0-8.0. Wood-based cellulose pulp, especially chemical birch pulp, was found advantageous for the present purposes. Chemical birch cellulose contains about 75% by weight of cellulose and about 25% by weight of hemicellulose. The wood may comprise or consist of birch. In one embodiment the wood-based cellulose comprises birch cellulose. In one embodiment the wood-based cellulose consists of birch cellulose.

In general cellulose which is used for preparing fibrillated celluloses may be unmodified or it may be chemically and/or enzymatically modified. In the present case it was found out that native (unmodified) cellulose, which is not chemically modified and/or enzymatically modified, was an advantageous choice for the present medical purposes. Usually native cellulose is fibrillated at lower concentrations and concentrated later. However it was found desirable for the present purposes to fibrillate the cellulose at the required concentration as modifying the hydrogel after preparation could destroy the properties desired for the present medical uses.

The native nanofibrillar cellulose maintains its original fibril length and thus has a high aspect ratio, which has impacts for example on the ability of the NFC to form a dense and stiff fibril network and biocompatible environment in the hydrogel.

The suspension of plant-based cellulose pulp used as the starting material may have a concentration (consistency) of the cellulose in the range of 3-12% by weight. The suspension may be provided directly from the manufacture of cellulose pulp, and it is a water suspension. The pulp may be bleached.

The suspension of cellulose pulp is examined and analyzed for several features, such as one or more, or all, of visual appearance, fiber length, FS5, and heterotrophs may be monitored by culturing. Other applicable analyses include determining one or more, or all, of properties such as dry matter content, SR°, pH, fiber dimensions, FS5, SEM/EDS, SOX-extraction, carbohydrates, solid samples, metals, ICP and endotoxins. In the present process it was found especially important to monitor the endotoxins during the whole production chain. If the pulp passes the predetermined limits for the properties, it can proceed to next process step.

The method may comprise washing the suspension of pulp. In one example the method comprises washing the cellulose with ultrapure water before pre-refining. During washing conductivity and pH are monitored. If necessary, acids, such as HCI and/or NaHCO₃, and/or bases such as NaOH are added to obtain a desired pH. During washing the pH may be lowered to pH 3 or less. The washing is continued and/or repeated until a conductivity at a desired range is obtained, such as 20 µS/cm or less, which may be measured with a conductivity meter from a water suspension. Conductivity at this range was found to facilitate pre-refining of the native pulp.

The concentration/consistency of the cellulose may be adjusted to a range of 8-12%, such as to about 10% by weight, after the washing is complete. This may require dewatering the pulp, which may be carried out with any suitable method.

The pulp may be applied to pre-refining after washing. Before pre-refining the pulp suspension may be analysed for one or more, or all, of features such as visual appearance, dry content, SEM/EDS, SOX-extraction, carbohydrates, solid samples, metals, ICP and/or endotoxins. The pulp shall pass the predetermined limits for said features in order to be used in the next step. Unless the limit is reached, the pulp is further processed, adjusted or discarded, depending on the feature. If the pulp passes the predetermined limits for the features, it can proceed to the pre-refining.

The pre-refining may be carried out at the pulp concentration obtained from the washing or adjusted after washing. A concentration of about 10% by weight may be used. The pulp is provided to pre-refining at the previously adjusted concentration. The pulp provided to the pre-refining may have an SR value of 30 or less, such as 22 or less, for example in the range of 14-22, or 20 or less, which indicates that it is unprerefined pulp.

The method comprises pre-refining the cellulose pulp suspension with a refiner to obtain a pre-refined cellulose dispersion having SR value 87 or more, such as SR value in the range of 87-95, or 87-93. SR (Schopper-Riegler) value measures drainability of a pulp suspension. The SR value can be determined by using a Schopper-Riegler Beating and Freeness Tester and/or by using ISO 5267-1:1999 standard.

Higher SR numbers means slower draining, which correlates with the refining degree. The present pre-refining provides such refined cellulose that does not block the disintegration device used in the disintegration, which would be often the case with native cellulose at relatively high concentrations. The pre-refining opens fibers but the obtained cellulose is not highly fibrillated which would make it difficult to handle in the next steps, such as in the dispersing step, and would prevent even distribution of water in the material.

The refining can be carried out with a suitable beater or mill, which preferably provides low-intensity and homogenous treatment.

In one embodiment the refiner is a mill with a roll with vertical cutting bars in a container, such as a PFI type of refiner mill. The method may comprise carrying out several or a plurality of, such as two or more, three or more, four or more or five or more, pre-refining runs, each with separate batch of pulp, and combining the obtained pre-refined batches. Carrying out the pre-refining in equivalent batches facilitated controlling the process and processing pure material with good quality.

The material obtained from the pre-refining is analysed for one or more, or all, of features such as visual appearance, dry matter content, SR°, pH, fiber dimensions, FS5, and endotoxins. The material shall pass the predetermined limits for said features in order to be used in the next step.

The method comprises dispersing the pre-refined cellulose pulp in water or other aqueous solution, or into water or other aqueous solution, to obtain a dispersion with a concentration of nanofibrillar cellulose in the range of 1.6-3.6% by weight. An aqueous solution comprising one or more salts and/or one or more buffering agents may be used, such as a physiological saline solution. The dispersing may refer to and/or include adjusting the concentration of the pre-refined pulp by using a dispersing device. The dry matter content and pH of the dispersed material may be analyzed and/or adjusted. Water of ultrapure grade may be added if it is necessary to dilute the pre-refined cellulose pulp dispersion. If necessary, the dispersion can be concentrated by filtering or by using any other suitable concentrating method. The dispersing may be carried out by any suitable dispersing device, such as a disperser, which preferably does not refine and/or fibrillate the cellulose.

In one example the dispersing the pre-refined cellulose into water or other aqueous solution before disintegrating is carried out at pH 8.5-9.0. However the pH may be adjusted to about 7, for example when a buffered solution is used, such as to 6.5-7.5. The method may include detecting the pH and/or adjusting the pH by adding a pH adjusting agent, which may be acid or base, as disclosed herein.

Next the method comprises disintegrating the pre-refined cellulose pulp dispersion by a disintegration device at said concentration to obtain medical grade nanofibrillar cellulose hydrogel. Mechanical disintegration of the cellulose raw material may be carried out with any suitable disintegration device such as a refiner, a grinder, a disperser, a homogenizer, a colloider, a friction grinder, a pin mill, a rotor-rotor disperser, an ultrasound sonicator, a fluidizer such as a microfluidizer, a macrofluidizer or a fluidizer-type homogenizer. The disintegration treatment is performed at conditions wherein water is sufficiently present to prevent the formation of bonds between the fibers. The disintegration may be called fibrillation, wherein fibrils are separated from fibrous cellulose.

As used herein, the term "fibrillation" generally refers to disintegrating fiber material mechanically by work applied to the particles, where cellulose fibrils are detached from the fibers or fiber fragments. The work may be based on various effects, like grinding, crushing or shearing, or a combination of these, or another corresponding action that reduces the particle size. The expressions "disintegration" or "disintegration treatment" may be used interchangeably with "fibrillation". The fiber material may be run through a disintegration device one or more times (passes), such as two or more times. This has an impact to the rheological properties of the obtained fibrillar cellulose, such as to the stiffness and viscosity.

A fluidizer was found to be a suitable disintegration device for preparing the present medical hydrogel, especially from native cellulose. A fluidizer, which may be a microfluidizer, may utilize one or more interaction chambers, and may include an inlet reservoir for the pulp dispersion, a pump for pressurizing the dispersion, wherein the pressurizer dispersion is conveyed to the interaction chamber, wherein in is disintegrated, and outputted from the chamber. The dispersion may be run through the fluidizer, or the interaction chamber(s), two or more times (passes), which are subsequent passes of the same material. The fluidizer may comprise two or more interaction chambers, which may differ in size, such as in chamber diameter, and/or which may differ in type. The interaction chambers may be single-slotted and/or multi-slotted. The interaction chambers contain channels, which may be microchannels. In one example the fluidizer comprises a first interaction chamber and a second interaction chamber having a smaller channel diameter compared to the first chamber. The method may include one or more passes through the first chamber, and one or more passes through the second chamber, and optionally one or more passes through a third chamber having a smaller channel diameter compared to the second chamber, so that a smaller channel size/diameter can be used as the fibrillation proceeds. This, as well as the pretreatment steps, enabled effectively fibrillating native cellulose at relatively high concentration of 1.5% by weight or more, 1.6% by weight or more, 1.7% by weight or more, 1.8% by weight or more or even 2% by weight or more, to obtain such high fibrillation degree, homogeneity of the material and desired properties which are required for the present medical uses in a body. Blocking of the interaction chambers was avoided with the present arrangement and method.

In one embodiment the disintegrating of the pre-refined cellulose pulp by a fluidizer comprises
- providing a microfluidizer comprising a first interaction chamber and a second interaction chamber as the disintegration device,
- carrying out a first disintegration run in a first interaction chamber, and
- carrying out one or more subsequent disintegration runs in a second interaction chamber having a smaller channel diameter compared to the first chamber. The subsequent disintegration runs are carried out for the material obtained from the first disintegration run.

The fluidizer may be equipped with or connected to one or more devices for monitoring the disintegration of the material, i.e. size reduction, such as devices arranged on-line, in-line, and/or off-line, such as with an optical device, for example with a laser diffraction analyzer, a dynamic light scattering analyzer, a turbidometer, or any other suitable device. The fluidizer may be controlled and adjusted in respect of process parameters, such as pressure, flow rate, selection of interaction chamber, processing time, number of disintegration runs (passes) and the like. The fluidizer may include control electronics for controlling the parameters and conditions.

The disintegration device may be a high-pressure homogenizer. In a homogenizer the fiber material is subjected to homogenization by an effect of pressure. The homogenization of the fiber material dispersion to nanofibrillar cellulose is caused by forced through-flow of the dispersion, which disintegrates the material to fibrils. The fiber material dispersion is passed at a given pressure through a narrow through-flow gap where an increase in the linear velocity of the dispersion causes shearing and impact forces on the dispersion, resulting in the removal of fibrils from the fiber material. The fiber fragments are disintegrated into fibrils in the fibrillating step. In order to obtain fibrillated cellulose the homogenization process is carried out with such adjustment of the device, such as the size of the gap, and with such process conditions, that the cellulose is disintegrated. A homogenizer can also be used for homogenizing cellulosic material without fibrillating, depending on said adjustment and process conditions.

The disintegration device may be a disperser having at least one rotor, blade or similar moving mechanical member, such as a rotor-rotor disperser, which has at least two rotors. In a disperser the fiber material in dispersion is repeatedly impacted by blades or ribs of rotors striking it from opposite directions when the blades rotate at the rotating speed and at the peripheral speed determined by the radius (distance to the rotation axis) in opposite directions. Because the fiber material is transferred outwards in the radial direction, it crashes onto the wide surfaces of the blades, i.e. ribs, coming one after the other at a high peripheral speed from opposite directions; in other words, it receives a plurality of successive impacts from opposite directions. Also, at the edges of the wide surfaces of the blades, i.e. ribs, which edges form a blade gap with the opposite edge of the next rotor blade, shear forces occur, which contribute to the disintegration of the fibers and detachment of fibrils.

The method comprises recovering the medical grade nanofibrillar cellulose hydrogel without adjusting the concentration, more particularly without actively adjusting the concentration, for example by adding or removing water. This refers to material obtained from the disintegration, such as from the fluidizer.

The process enables obtaining nanofibrillar cellulose hydrogel having a final concentration of nanofibrillar cellulose in the range of 1.4-3.4% by weight, such as 1.45-3.0% by weight. It was found out that during disintegration the concentration of the cellulose slightly decreased, such as by 0.2-0.3 percentage units. In most uses a concentration of nanofibrillar cellulose in the range of 1.5-3.2% by weight, or in the range of 1.5-3.0% by weight was found most suitable. In some examples it may be desired to use slightly higher concentrations in medical applications, such as a final concentration of nanofibrillar cellulose of 1.6% or more, 1.7% or more, 1.8% or more, even 2.0% or more. Therefore for certain uses a concentration in the range of 1.6-3.2% by weight, 1.7-3.2% by weight, 1.8-3.2% by weight or even 2.0-3.2% by weight may be desired. The final concentrations may refer to concentrations after autoclaving.

In the disintegration process the cellulosic dispersion may be monitored and/or analyzed for one or more features or parameters, such as pH, dry matter content, rheological properties and turbidity. This may be carried out from samples taken from the process and/or by using one or more suitable monitoring devices, which may be connected in-line or on-line.

After disintegration and/or prior to autoclaving the formed hydrogel may be analyzed for one or more features or parameters, such as visual appearance, pH (ISO 6588-1:2020), dry matter content (Mod. ISO 638-2 (2021)), rheological properties, turbidity, endotoxins, bacteria and fungi (aerobic and anaerobic).

The process or parts thereof is preferably carried out at clean production room conditions, such as according to ISO 14644 standard, which may include controlled relative humidity percentage (RH%), temperature, pressure difference, airborne particles, cleaning and its control (clean cards), restricted access and clothing. The preparation method is preferably carried out using pure, clean and/or sterilized materials, reagents, vials, syringes, containers, and devices. Any added water is preferably ultrapure grade.

The disintegration is carried out until desired properties of the nanofibrillar cellulose are obtained, which may require carrying out a number of disintegration runs and/or adjusting device setup and/or process conditions.

In one embodiment the pre-refined cellulose pulp is disintegrated until the obtained nanofibrillar cellulose provides a storage modulus in the range of 1-40 Pa determined by a rotational rheometer using plate geometry at a consistency of 0.5% by weight in aqueous medium at 22±1°C. In one embodiment the pre-refined cellulose pulp is disintegrated until the obtained nanofibrillar cellulose provides a storage modulus in the range of 0.7-20 Pa determined by a rotational rheometer using vane geometry at a consistency of 0.5% by weight in aqueous medium at 22±1°C.

In one embodiment the pre-refined cellulose pulp is disintegrated until the obtained nanofibrillar cellulose provides a zero-shear viscosity in the range of 1500-50000 Pa·s and a yield stress in the range of 2-15 Pa, determined by a rotational rheometer using plate geometry at a consistency of 0.5% by weight in aqueous medium at 22±1°C.

In one embodiment the pre-refined cellulose pulp is disintegrated until the obtained nanofibrillar cellulose has a number-average diameter of fibrils and/or fibril bundles of 100 nm or less, such as in the range of 1-100 nm, for example in the range of 1-50 nm or 10-50 nm.

For the uses involving inserting the hydrogel into a body, such as for supporting tissues and/or providing filler, it is not necessary, or even desired, to include active agents to the hydrogel, such as pharmaceuticals or other active agents. For these purposes there is no need to provide the hydrogel in such form that provides a specific release profile of any active agent, but instead the hydrogel can be prepared to exhibit properties, which support the present uses and which properties are maintained for a prolonged time. It is not desired that the hydrogel provides agents to the body in the target, or that the content of the hydrogel changes over time.

In its simplest form the medical grade nanofibrillar cellulose hydrogel consists of nanofibrillar cellulose and water or aqueous saline and/or buffer solution. The medical grade nanofibrillar cellulose hydrogel may consist of only nanofibrillar cellulose and water or aqueous saline and/or buffer solution, such as physiological saline. The hydrogel, for example as an implant or a filler, may be used as a supportive "empty" hydrogel as such for purposes. One example is a dermal filler, for example for facial reconstruction of congenital deformity or after trauma or disease, female pelvic organ prolapse, breast reconstruction and augmentation, hernia repair sphincter repair and incontinence. Such an empty hydrogel does not include active agents, such as active pharmaceutical ingredients (API) , such as drugs, other bioactive substances, for example enzymes, vitamins, phytochemicals, and other bioactive compounds or the like, which can influence an organism, tissue or cell.

However the hydrogel may include non-active agents, such as one or more salts, buffering agents and/or the like, such as inorganic salts, inorganic ions and/or buffering agents, which can provide suitable physiological conditions in the body and/or adjust the hydrogel to be compatible with the body, with the tissue or with the target, but which are preferably not intended to induce a bioactive effect in the body, in the tissue and/or in the target. In one example the medical hydrogel is based on physiological saline solution. Physiological saline or the like solution may facilitate the stability of the hydrogel in the target, and help avoiding undesired osmotic effects. Physiological saline may be an approximately 0.9% by weight aqueous solution of sodium chloride. The pH of the aqueous solution, such the buffer, may be about 7, such as in the range of 6.5-7.5. Multivalent metal cations may be used for ionically (non-covalently) crosslinking the NFC, such as cations of calcium, barium, magnesium, zinc, aluminium, gold, platinum and titanium, which can be used for increasing the stiffness of the hydrogel. The non-active agents may be added in total amounts of 2.0% by weight or less, or 1.0% by weight or less or 0.5% by weight or less, such as in the range of 0.1-2.0% by weight, or 0.1-1.0%, calculated from the dry content of the hydrogel. The possible non-active agents may be provided in the step of dispersing the pre-refined cellulose pulp.

In one embodiment the medical grade nanofibrillar cellulose hydrogel comprises no active agents, such as therapeutic agents or bioactive agents. Correspondingly the preparation method does not comprise including such active agents, i.e. the preparation may be carried out in the absence of active agents. The obtained and preferably provided medical grade nanofibrillar cellulose hydrogel comprises no active agents. The method may comprise recovering, and preferably applying into a container, syringe or other applicator and/or packing, the medical grade nanofibrillar cellulose hydrogel from the disintegrating device without including or adding active agents.

In other examples the medical grade NFC hydrogel may include active, or bioactive, agents, such as therapeutic agents, such as drug compounds, biological therapeutic agent(s), therapeutic peptides and proteins, extracellular vesicles, and/or cells. Such agents may not be desired in uses involving inserting the hydrogel into a body.

In some examples the method comprises adding one or more further substances to the obtained nanofibrillar cellulose hydrogel. The substances may be bioactive substances, such as therapeutical agents, or structural substances, such as other polymers and/or gel-forming agents, such as hyaluronic acid, or cross-linking agents, such as divalent metal cations.

The nanofibrillar cellulose may be the only matrix material in the hydrogel, such as the only polymeric material and/or the only cellulosic material. However, in some examples it is also possible to include one or more other polymeric materials in addition to NFC, such as nanocrystalline cellulose, hyaluronan, hyaluronic acid and its derivates, peptide-based materials, proteins such as elastin-like protein, elastin-like protein-hyaluronic acid (ELP-HA), other polysaccharides such as alginate or polyethylene glycol. Compositions forming a semi-interpenetrating network (semi-IPN) may be obtained, where nanofibrillar cellulose provides structural stability. The content of the other polymeric materials in the total composition as dry weight may be in the range of 20-80% (w/w), such as 40-60% (w/w), or 10-30% (w/w) or 10-20% (w/w). For example hyaluronic acid or hyaluronan or derivatives thereof may be included to provide properties useful for example in cartilage repair applications because of their properties.

The NFC hydrogel may be provided in a suitable container, such as a vial, bottle, tube or the like, or in a syringe or other application device. In one embodiment the NFC hydrogel in provided in a syringe. The NFC hydrogel and/or the container may be sterilized and/or provided as sterilized, such as by autoclaving, by irradiating, such as by gamma irradiation, or by using any other applicable sterilization method. The container may be sealed or packed in a sealed sterile package.

The present disclosure provides a medical grade nanofibrillar cellulose hydrogel obtained by the method disclosed herein.

The present disclosure provides a medical grade nanofibrillar cellulose hydrogel comprising plant-based, such as wood-based, nanofibrillar cellulose hydrogel having a concentration of the nanofibrillar cellulose in the range of 1.4-3.4 by weight, such as 1.45-3.0% by weight, the nanofibrillar cellulose having a number-average diameter of fibrils and/or fibril bundles of 100 nm or less, and a storage modulus in the range of 1-40 Pa determined by a rotational rheometer using plate geometry at a consistency of 0.5% by weight in aqueous medium at 22±1°C. Alternatively the storage modulus may be presented with values measured by using vane geometry, which is in the range of 0.7-20 Pa. The nanofibrillar cellulose is preferably chemically and/or enzymatically unmodified. The concentration of the nanofibrillar cellulose is preferably unchanged after manufacture, such as the nanofibrillar cellulose is undiluted and/or unconcentrated.

In one embodiment the nanofibrillar cellulose comprises chemically and enzymatically unmodified nanofibrillar birch cellulose. Preferably the nanofibrillar cellulose consists of chemically and enzymatically unmodified nanofibrillar birch cellulose. The birch nanofibrillar cellulose may be the only cellulosic material in the medical grade nanofibrillar cellulose hydrogel, and it may be the only polymeric and/or fibrillar material in the medical grade nanofibrillar cellulose hydrogel. The medical grade nanofibrillar cellulose hydrogel preferably does not contain any fibrous material, such as fibrous cellulose.

### Use of the nanofibrillar cellulose

The present medical grade NFC hydrogels may be used externally or internally. External uses include treatment of wounds or other damages, such as topical uses, for example applying the hydrogel onto skin or other tissue. The external uses may include for example first aid uses of a patient, wherein the medical grade hydrogel may be applied to immediately cover, protect and moisturize the damaged area. The hydrogel can provide efficient protection until the patient is brought to further medical care, wherein the hydrogel can be simply removed without leaving any residues to the damaged area.

The hydrogels may be mouldable, such as hand-mouldable, or they may be injectable, which properties are useful in external uses. The hydrogel may be applicable from a syringe or other application device, or from a sealed package, directly to the damaged area. Examples of wounds include deep wounds or dermis and/or below tissue(s), and burns. The stiffness of the present hydrogels provides specific mouldability, which enables quickly and safely covering the damaged area, such as in the case of emergency, such as in the case of a deep wound. The present medical grade NFC hydrogels help preventing bleeding and provide such conditions in the wound that can prevent further damages and contamination. The shear-thinnability of the hydrogel, especially at an increased temperature which is obtained when the hydrogel is hand-moulded, helps moulding the hydrogel onto the wound or other damaged area. The hydrogel may be moulded after applying from a package or applicator device to the damaged area, so that a sterile part of the hydrogel covers the damaged area and remains thereon, and the hydrogel is moulded from above and/or from sides, for example to spread and to better set onto the damaged area, and to adhere to the damaged area. Therefore no contaminants can enter the damaged area. An applicator device may be a syringe or other suitable applicator device, which may have a specific nozzle for obtaining a specific shape of the applied hydrogel, for example a sheet-like or otherwise flat shape, which is useful for instantly covering larger areas and which decreases the risk of contaminating the hydrogel at the damage site during manual molding.

The nanofibrillar cellulose hydrogel may be used internally in a body, for example as implantable material, which refers to materials which may be injected, implanted or inserted into a subject's body, with any suitable method, such as by injecting, by surgery or other invasive methods. Injection is preferred for the present uses. Inserting material into a body cavity with non-invasive method may be excluded. In such uses the medical grade properties are especially pronounced, as any material entering a body must be absolutely safe in many respects.

In the present uses and methods the medical grade nanofibrillar cellulose hydrogel remains in the body for a prolonged time period, and they may be provided for use as implantable material in a body for 14 days or more, such as 28 days or more, 2 months or more, 3 months or more or 6 months or more. As evidenced experimentally, the medical grade nanofibrillar cellulose hydrogels are well tolerated and maintain their form in the body, so they are suitable for such long-term use.

The present applications include uses and methods involving placing the material into a body, such as into a target in a body, for example through a skin or other surface tissue, for example as an implant or a part thereof, and/or as a filler, for example as a dermal filler, as viscoelastic supplementation or as a barrier. The present material is non-absorbable, which enables providing non-absorbable implants, fillers, supplementations and barriers.

The present medical grade hydrogel allows for example a targeted injection of the hydrogel into a bone cavity, thus favouring filling and/or consolidation, without the risk of constraining any nerve, and/or any other soft portion, that can be present in the vicinity of the injection site.

As used herein, the term "dermal filler" is used to denote materials which are placed under the skin or in the dermal layers to correct or alter soft tissue. Dermal fillers may be used to augment tissues which may suffer loss of volume due to lipodystophy, pathological atrophy, trauma, or as a cosmetic agent, for example to address perceived defects and the effects of aging.

The present medical grade nanofibrillar cellulose hydrogel can be used in methods including inserting the hydrogel into a body. The inserting may comprise implanting, injecting or inserting surgically. Injecting enables inserting the material into a desired target without need to surgically treat the body, such as skin and/or other tissues. The hydrogel may be injected into a desired form and in desired amounts, and during the injection in some cases it is possible to see how the material forms under skin, so the present hydrogels enable controlling the injecting. This is especially advantageous for example in aesthetical applications, such as in plastic surgery wherein the hydrogel is used as a filler.

In one example a method for treating a subject in need of treatment of body with implantable material, which may be human or non-human animal, comprises
- preferably recognizing a subject in need of treatment of body with implantable material,
- providing the medical grade nanofibrillar cellulose hydrogel,
- injecting, implanting or inserting the medical grade nanofibrillar cellulose hydrogel into the subject's body, preferably to provide a tissue-supporting and/or filling implant, or a filler, such as a dermal filler, in the body. The medical grade nanofibrillar cellulose hydrogel may be injected or inserted into a target in the body. The target may have been recognized and/or selected after examining the subject, and recognizing a need for treatment, such as providing implant, filler and/or viscoelastic supplementation to the target. The target may be or comprise skin, such as epidermis, dermis or subcutaneous tissue, or a junction thereof, or muscle, fat tissue, cartilage, bone, or a junction of two or more thereof, or a site on close proximity to one or more thereof. The tissue discussed herein may comprise one or more of aforementioned.

The treatment may be a medical treatment, which preferably aims for treating, such as curing or alleviating, a medical condition and/or symptoms thereof, such as a disease, a disorder, a condition originating from an accident, or the like. Implants, such as tissue-supporting implants, or tissue supporting fillers, or other filler, or viscoelastic supplementation may be used in medical treatments.

The treatment may be an aesthetical treatment, which may ne medical or non-medical, and which may include plastic surgery and the like operation, for example plastic surgery, which is a surgical specialty involving the restoration, reconstruction, or alteration of the human body. Plastic surgery may be reconstructive surgery and/or cosmetic surgery. Reconstructive surgery aims to reconstruct a part of the body or improve its functioning. Cosmetic (or aesthetic) surgery aims at improving the appearance of it.

The present disclosure provides a medical grade nanofibrillar cellulose hydrogel for use as injectable insertable and/or implantable material in a body. The implantable material may be supporting material, such as tissue-supporting material, which may refer to mechanically supporting the tissue(s).

One embodiment provides the medical grade nanofibrillar cellulose hydrogel for use as an implant, such as a tissue-supporting implant in a body. In one example the treatment comprises injecting, implanting or inserting the medical grade nanofibrillar cellulose hydrogel as a tissue-supporting implant into a target in the subject's body.

One embodiment provides the medical grade nanofibrillar cellulose hydrogel for use as a filler, such as a tissue-supporting filler in a body. In one example the treatment comprises injecting, implanting or inserting the medical grade nanofibrillar cellulose hydrogel as a tissue-supporting filler into a target in the subject's body.

One embodiment provides the medical grade nanofibrillar cellulose hydrogel for use as a dermal filler in a body. In one example the treatment comprises injecting, implanting or inserting the medical grade nanofibrillar cellulose hydrogel as a dermal filler into a target in the subject's body. The target may comprise any suitable part of the body, such as face (facial target), head, neck, chest, breast, arm, hand, fingers, abdominal area, pelvis area, back, buttocks, genital area and/or genitals, legs, feet and/or toes. In such cases injecting into the target may cause visible effect, which may be augmentation, shaping, highlighting and/or other visible effect.

The dermal filler may be augmenting filler, such as for augmentation of facial areas, such as lips, cheeks or chin, or back of the hand, or the like. The dermal filler may be used for restoration and correction of signs of facial fat loss, or other facial defects or fat loss in other parts of the body. The dermal filler may be used for correction of contour deficiencies, such as wrinkles and acne scars. The dermal filler may be also used for other body contour and enhancement purposes, such as to increase breast size, to increase size of the buttocks, to increase fullness of the feet, to implant into a bone, tendon, ligament or muscle and/or to inject the glabella (area between eyebrows), nose, periorbital area (around the eyes), forehead, or neck. Some of the mentioned fillers may be also considered as implants.

Viscosupplementation, or viscoelastic supplementation, may be used for treatment of arthritis, such as osteoarthritis, or other joint conditions. The joint may be a natural joint or an artificial joint. Viscoelastic supplementation may comprise injecting the present hydrogel into diarthrodial joints. The aim is to restore the rheological properties of the synovial fluid, thereby producing mechanical, analgesic, anti-inflammatory and chondroprotective effects. The present NFC hydrogels were found to exhibit suitable rheological properties for such use, and the material is well tolerated by the body. The present NFC hydrogels can be used to replace presently used hyaluronic acids and derivatives thereof in viscosupplementation.

Viscoelastic supplementation may be used for example for palliative treatment for knee osteoarthritis because of ease of application and theoretical ability to relieve symptoms by restoring and replenishing hyaluronate component into the knee joint.

One embodiment provides the medical grade nanofibrillar cellulose hydrogel for use as a viscoelastic supplementation for treatment of arthritis or other joint conditions. In one example the treatment comprises injecting, implanting or inserting the medical grade nanofibrillar cellulose hydrogel as viscoelastic supplementation into a target in the subject's body. The target may be a joint or another target, which suffers from arthritis or other joint condition.

The nanofibrillar cellulose hydrogel may be used as a barrier agent in surgery. Surgical post-operative adhesions can lead to serious clinical complications. Barrier agent may be used for the prevention of post-operative adhesions, such as for reducing abnormal internal scarring following surgery by separating the internal tissues and organs with the barrier while they heal. The present hydrogels may be inserted in the target during the surgery to form a barrier, i.e. before wound closure, or the hydrogel may be inserted, such as injected, to the target after the surgery, for example after wound closure. It is also possible to insert a hydrogel or an implant during surgery. A specific applicator device with a nozzle for obtaining a sheet-like hydrogel form may be used.

One embodiment provides the medical grade nanofibrillar cellulose hydrogel for use as a barrier agent in surgery. In one example the treatment comprises injecting, implanting or inserting the medical grade nanofibrillar cellulose hydrogel as a barrier agent in surgery into a target in the subject's body. The target may be any tissue or combinations thereof discussed herein under the surgery. A barrier is formed in the target, which may separate two areas, such as two tissues, which may be similar or different, and which may prevent adhesion of the tissues together.

Disclosed is an implant comprising the medical grade nanofibrillar cellulose hydrogel. The implant may be insertable or inserted in a body, as disclosed herein, for example by injecting, i.e. an injectable or injected implant, or it may be surgically inserted. The implant may be formed by injecting the hydrogel into a target. Similarly, disclosed is a filler or a barrier comprising the medical grade nanofibrillar cellulose hydrogel, which may be insertable or inserted in a body, i.e. an injectable or injected, or surgically inserted, filler or barrier. The medical grade nanofibrillar cellulose hydrogel may be provided in an application device, such as a syringe, which may be equipped with an injection needle, wherein the hydrogel is ready to be used, i.e. injectable or insertable.

Disclosed is use of birch cellulose, such as birch pulp, for manufacturing the medical grade nanofibrillar cellulose hydrogel. The present disclosure also provides use of nanofibrillar cellulose for preparing any of the products disclosed herein and for any of the uses disclosed herein.

### Examples

### Example 1: Preparation and characterization of medical grade hydrogels

A variety of batches of chemically and enzymatically unmodified birch NFC with different concentrations were prepared with the present method by first pre-refining with a refiner mill, adjusting the concentration and subsequently disintegrating with a microfluidizer with several number of passes until desired properties of the hydrogels were obtained. The hydrogels were obtained directly from the disintegration without adjusting the concentration or adding any additional substances, and they were autoclaved.

The hydrogels (originally 1.5%, 2% and 3% by weight) were characterized as diluted to 0.5% by weight concentration for rheological properties by using RH-1 rotational rheometer with either vane geometry or 20 mm plate geometry at 22°C, and a specific measuring program. Brookfield viscosity was measured with a Brookfield viscometer according to modified SCAN-P 50:84. Properties from a variety of samples and production batches are summarized and presented in Table 1. Measurements from latest samples are presented in parenthesis.

**Table 1.**

| **Hydrogel concentration** | **1.5%+0.05** | **2.0%+0.17** | **3.0%+0.27** |
|---|---|---|---|
| Zero shear viscosity Pa·s (vane geometry) | 1362-2074 (1362-1775) | 787-1237 | 380-752 |
| Zero shear viscosity Pa·s (plate geometry) | 2199-40337 (5782-10035) | 6138-8254 | 1630-5967 (5072-5967) |
| Storage modulus Pa (vane geometry) | 2.12-6.49 (2.12-3.13) | 1.35-1.94 | 0.76-1.55 |
| Storage modulus Pa (plate geometry) | 8.22-30.80 (8.22-11.79) | 8.56-10.11 | 3.21-7.68 |
| Fracture strength Pa (yield stress) (vane geometry) | 0.79-1.99 (0.79-1.58) | 0.79-1.00 | 0.25-0.79 |
| Fracture strength Pa (yield stress) (plate geometry) | 0.79-6.27 (0.79-3.07) | 2.45-3.25 | 0.50-2.51 (0.50-1.98) |
| Loss modulus Pa (vane geometry) | 0.35-1.14 (0.35-0.42) | 0.21-0.27 | 0.15-0.31 |
| Loss modulus Pa (plate geometry) | 1.02-3.77 (1.02-1.93) | 1.31-1.62 | 0.56-0.98 |
| Brookfield viscosity mPa·s (10 rpm) | 8614-11180 (7169-10645) | 6955-8828 | 6715-9646 |

Aerobic bacteria (Petrifilm), anaerobic bacteria (Petrifilm), aerobic yeasts and mold (Petrifilm), anaerobic yeast and molds (Petrifilm) were all 0 CFU/g measured with modified SFS-EN ISO 8199. Endotoxin (LAL kinetic chromogenic) was 2.07 EU/ml (Ph. Eur. 2.6.14). pHs of the hydrogels were in the range of 6.2-7.8, mostly in the range of 6.7-7.1.

### Example 2: Animal tests

The birch-based native (chemically and enzymatically unmodified) nanofibrillar cellulose (nNFC) prepared as disclosed herein was used in tests. The nNFC was manufactured under quality management system in compliance with the requirements of the international standard EN ISO 13485:2016 "Medical devices. Quality management systems. Requirements for regulatory purposes".

The product was slightly opaque white semi-solid homogenous hydrogel without visible fibers or particles. The physical properties and microbial contamination were analyzed, and the product was sterile (Ph. Eur. 2.6.1) containing bacterial endotoxins ≤ 5.5 EU/g (Ph. Eur. 2.6.14). The product was packed in 10 ml syringes sterilized by autoclaving.

### Animal tests

The aim of the study was to clarify the nNFC (native nanofibrillated cellulose) hydrogel stability as a supportive implant in the subcutaneous tissues and possible foreign tissue reaction around the injected test item. The foreign material tissue reaction evaluation based on the macroscopic observations.

### MATERIALS AND METHODS

### Study Design

The study was performed according to Table 2 below.

**Table 2.**

| Rat ID # | Treatment | | Necropsy (Blood sampling and macroscopical evaluation) |
|---|---|---|---|
| | Left flank | Right Flank | |
| 1-6 | 200 µl of nNFC s.c. | 200 µl of nNFC s.c. | Day 28 |

### Test System

Species and strains: Rat Hsd:SD/TY
Origin of the animals: University of Turku, Central Animal Laboratory, PharmaCity Unit
Quality: SPF (Specific Pathogen Free)
Acclimatization: No adaptation period was needed because the animals were previously in the same facility.
Animal identification: The animals were individually identified by tail numbering and the tail marking was maintained throughout the study. Cages were respectively marked with cage cards indicating study number, animal numbers and tail markings, sex, strain and in life study period.
Number of animals in the study at the start of the study: 6 male rats

Weight and age at the start of the study (the first dosing day)

| | |
|---|---|
| Weight | 232 g (217 g - 248 g; SD 12.9 g) |
| Age | 8 weeks |

Randomization and grouping: There was no need for randomization or grouping.

### Housing Conditions

The animals were housed in room number 317 (BioCity animal unit, University of Turku). The room temperature was 21±3°C, relative humidity mainly 40-60%. Lighting was artificial, 12 h light and 12 h dark (dark 17:00-07:00).
Animal care: The animals were taken care of according to the standard operating procedures of Animal Laboratory.
Number of animals per cage: 3 animals/cage

### Test Item

1.5% by weight sterile nNFC was used as a test item. The test items were stored at RT in a sealed container and protected from light.

The nNFC hydrogel was available as a ready use formulation. The gel was delivered in 10 ml plastic syringes. The gel was transferred aseptically to a new sterile syringe (Terumo^{®} 2.5 ml, lot 171209L, Exp.Date 2022-11) to achieve better injection accuracy (0.2 ml) just prior to injection.

### Experimental procedures

Weighing: The animals were weighed on study days 0, 7, 14, 21 and before necropsy.

Dosing: The animals were under anesthesia (Isoflurane 3%) during the s.c. injection. Before the injection, the fur was clipped (1 cm x 1 cm) using B Braun Isis Cordless Clipper and skin disinfected with 70% ethanol.

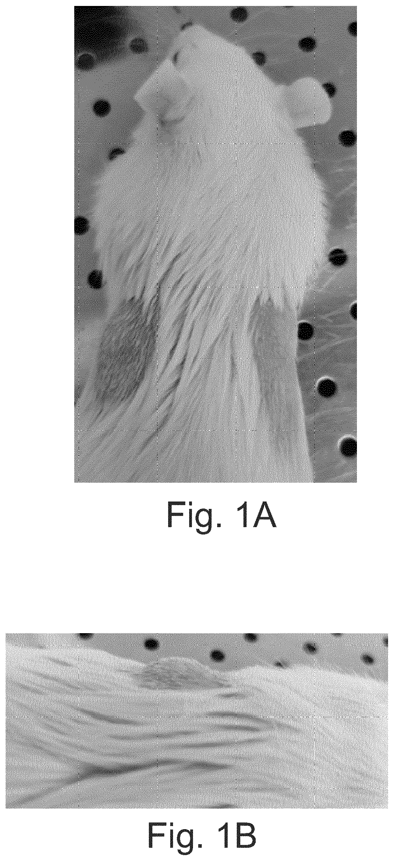

Test item was injected 0.2 ml (left flank) and 0.2 ml (right flank)/rat using Medoject^{®} 0.8x40 mm 21G×1½" needle (lot 170806, Exp.Date 2022-07). Nodules were clearly observed after gel injection (figure 1). The total number of injection sites was 12.

Figure 1A shows gel nodules immediately of the injection (both side). Figure 1B shows a gel nodule immediately of the injection.

The injected gel weight per rat was between 170 mg and 237 mg (average 193 mg, STDEV 16.7 mg). The injected volume is estimated by weighing the weight of 17 x 0.2 ml injections (sampling was done in the same way as sc. injection was performed).

Clinical examination: The animals were observed twice a day (morning and afternoon) for general well-being, except at weekends once a day.

The injection site, nodule, were measured (length and width and height whenever possible) using digital calliper immediately after injection (study day 0), and at study days 1, 7, 14 and 28.

Necropsy: The animals were euthanized by CO₂ 28 days after dosing and macroscopical evaluation of the tissue reaction around the gel was performed. The gel was removed and weighted.

Sampling: Blood (plasma) samples for possible further analysis at necropsy (D28). The results of the blood samples are not included in this report.

### EXPERIMENT TERMINATION AND SAMPLING

Animals were necropsied according to study design on day 28 using CO₂. Terminal samples were taken from the heart puncture (2 ml for 400-500 µl of plasma).

The blood was centrifuged for plasma separation, within 30 min (15 min, 2700 G, RT). The plasma samples were transferred into plastic tubes and they were frozen and stored at -20°C.

A description of any macroscopic abnormalities at the injection sites was recorded and the gel nodules were measured (external measurements, whenever possible). The residual gel was carefully removed from the subcutaneous tissue and weighed.

### RESULTS

### Clinical signs and mortality

All animals surveyed in good condition and any abnormal clinical signs were not observed during the experiment period.

### Weight

Table 3 presents the animals weights and weight gain during the experiment period.

**Table 3. Individual Animal Data; body weights of rats during the study. (* Weight gain between at study day 0 and at necropsy.**

| **#** | **Study Day** | | | | | **Weight gain^{(*}** |
|---|---|---|---|---|---|---|
| | **0** | **7** | **14** | **21** | **28** | |
| 1 | 217.22 g | 263.71 g | 282.54 g | 298.89 g | 319.91 g | +103 g |
| 2 | 248.08 g | 294.94 g | 324.46 g | 339.79 g | 363.72 g | +116 g |
| 3 | 242.13 g | 283.25 g | 308.70 g | 335.51 g | 358.19 g | +116 g |
| 4 | 239.12 g | 284.83 g | 312.68 g | 333.53 g | 359.03 g | +120 g |
| 5 | 218.04 g | 252.67 g | 275.77 g | 297.01 g | 318.21 g | +100 g |
| 6 | 230.00 g | 276.83 g | 309.45 g | 332.95 g | 352.20 g | +122 g |

Table 4 presents the measure results as a surface (mm²) and volume (mm³). Not all nodules/depots could be measured at all time points. The average depot/nodule sizes have been calculated as a mm² and mm³ at each measure time points.

**Table 4. The average nodule sizes at the study days 0 (immediately after the injection) and at study days 1, 7, 14 and 28.**

| **Study Day** | **Average depot size** | | | |
|---|---|---|---|---|
| | **mm²** | **STDEV** | **mm²** | **STDEV** |
| **0** | 182 | 13.0 | 1480 | 215.3 |
| **1** | 189 | 37.0 | 1356 | 446.5 |
| **7** | 152 | 16.6 | 1032 | 256.4 |
| **14** | 151 | 31.4 | 983 | 373.5 |
| **28** | 193 | 26.2 | 1248 | 359.3 |
| **Change between D0 and 28** | +11 | | -232 | |

Table 5 shows the weight of the removed gel depots after 28 days of injection.

**Table 5. The weight of the depots, when removed from a subcutaneous tissue at the end of the experiment period, at study day 28.**

| **Rat #** | **Depot weight (g)** | | **Notes** |
|---|---|---|---|
| | **Right flank (A)** | **Left flank (B)** | |
| **1** | 94.1 | 97.9 | The gel nodules were not measurable (dimensions) |
| **2** | 76.6 | 143.4 | - |
| **3** | 153.2 | 142.6 | - |
| **4** | 100.2 | 80.8 | - |
| **5** | 97.9 | 122.2 | - |
| **6** | 27.5 | 132.0 | A: The gel nodule was not measurable (dimensions) |
| **Average** | 105.7 mg | | - |
| **STDEV** | 35.511 mg | | - |

### Macroscopical observations

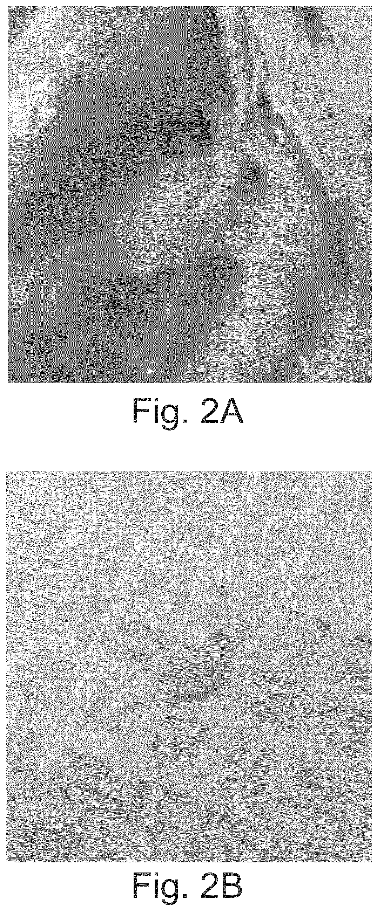

A clear injection site was visible in all the injection sites when the skin was opened. The macroscopical observations are presented on Table 6 and typical macroscopical observations is presented in Figures 2A and 2B. Figure 2A shows macroscopical observation on the NFC injection sites 28 days after injection (1A). Figure 2B shows the NFC hydrogel after removing from subcutaneous tissue for weighing.

All injection sites were clearly observable and the gels visible at necropsy, 28 days after injection. Any abnormal tissue reactions were not observed macroscopically in injection sites at time point 28 days. The capsule formation was not observed macroscopically.

**Table 6. Individual animal data; macroscopical observations at necropsy 28 days after the injection. NOA = Not observed abnormal tissue reaction.**

| **TI** | **Time point** | **Animal ID** | **Abnormal Macroscopical observations** |
|---|---|---|---|
| | | | A = Right B = Left |
| nNFC | D28 | 1 | A: a clear injection spot (depot) visible, inside the subcutaneous fat, NOA |
| | | | B: a clear injection spot (depot) visible, on blood vessels on the depot, NOA |
| | | 2 | A: a clear injection spot (depot) visible, inside the subcutaneous fat, NOA |
| | | | B: a clear injection spot (depot) visible, inside the subcutaneous fat, NOA |
| | | 3 | A: a clear injection spot (depot) visible, blood vessels on the depot, NOA |
| | | | B: a clear injection spot (depot) visible, NOA |
| | | 4 | A: a clear injection spot (depot) visible, NOA |
| | | | B: a clear injection spot (depot) visible, inside the subcutaneous fat, NOA |
| | | 5 | A: a clear injection spot (depot) visible, NOA |
| | | | Right: a clear injection spot (depot) visible, NOA |
| | | 6 | A: a clear injection spot (depot) visible, NOA |
| | | | B: a clear injection spot (depot) visible, inside the subcutaneous fat, NOA |

### DISCUSSION

Six rats were injected with nNFC gel in both flanks (s.c.; 2 x 0.2 ml). The outer dimensions of skin nodules caused by nNFC gel injection were measured immediately after injection (D0) and on D1, D7, D14 and D28. The animals were euthanized, the tissue reaction around the gel was evaluated macroscopically, the gel residues were removed from the subcutaneous tissue and weighed on the day 28 of the study.

Animals survived in good conditions through the study. Abnormal clinical signs were not observed during the study and the weight gain was normal in all animals. The nodule increase was not observed during the experiment and this indicated normal mild foreign tissue reaction of the nNFC gel. If a severe foreign tissue reaction exists, the nodule size would increase clearly between study day 7 and 14. Based on the depot/nodule measure data (surface area and volume data), the gel was stable in the subcutaneous tissue of the rat. The average surface area was a little bit larger after day 28 experiment period but the volume a little bit smaller. This suggests that the gel depot loses its height but increases its surface area; the shape of the gel changes a little bit under the skin, in subcutaneous tissue.

Based on the measurement data can be evaluate stability of the nNFC gel; the nNFC gel is quite stabile in subcutaneous tissue.

All injected gel depots were found at study day 28.

Based on the depots weight, the gel lost 54% of weight during 28-day experiment period. This is a normal tissue reaction because the water is a main element in the nNFC gel; the water is slowly absorbed into the tissue surrounding the gel.

However, the nNFC seems to be stable in injection site and there was no change of location of the gel after injection.

Based on the macroscopical observations, the nNFC gel does not affect the severe foreign tissue reaction and clear capsule formation was not observed. The nNFC gel was easy to remove from the subcutaneous tissue of the rats and the interface between the tissue and the gel was clear.

In conclusion, nNFC gel 1.5% by weight was clearly found at the injection site 28 days after injection without macroscopically visible foreign tissue reaction.

## Claims

1. A medical grade nanofibrillar cellulose hydrogel comprising plant-based, such as wood-based, chemically and enzymatically unmodified nanofibrillar cellulose hydrogel having a concentration of the nanofibrillar cellulose in the range of 1.4-3.4% by weight, such as 1.45-3.0% by weight, the nanofibrillar cellulose having a number-average diameter of fibrils and/or fibril bundles of 100 nm or less, and a storage modulus in the range of 1-40 Pa, such as 8-35 Pa, determined by a rotational rheometer using plate geometry at a consistency of 0.5% by weight in aqueous medium at 22±1°C and/or a storage modulus in the range of 0.7-20 Pa determined by a rotational rheometer using vane geometry at a consistency of 0.5% by weight in aqueous medium at 22±1°C.

2. The medical grade nanofibrillar cellulose hydrogel of claim 1, comprising bacterial endotoxins 5.5 EU/g or less determined according to Ph. Eur. 2.6.14.

3. The medical grade nanofibrillar cellulose hydrogel of claim 1 or 2, wherein the nanofibrillar cellulose comprises chemically and enzymatically unmodified nanofibrillar birch cellulose, preferably wherein the nanofibrillar cellulose consists of chemically and enzymatically unmodified nanofibrillar birch cellulose.

4. The medical grade nanofibrillar cellulose hydrogel of any of the preceding claims, wherein the nanofibrillar cellulose has a zero-shear viscosity in the range of 1500-50000 Pa·s and a yield stress in the range of 0.5-10 Pa, determined by a rotational rheometer using plate geometry at a consistency of 0.5% by weight in aqueous medium at 22±1°C.

5. The medical grade nanofibrillar cellulose hydrogel of any of the preceding claims, comprising no active agents, such as therapeutic agents or bioactive agents.

6. The medical grade nanofibrillar cellulose hydrogel of any of the claims 1-5 for use as implantable material in a body.

7. The medical grade nanofibrillar cellulose hydrogel of claim 6 for use as implantable material in a body for 28 days or more, or for 2 months or more.

8. The medical grade nanofibrillar cellulose hydrogel of claim 6 or 7 for use as a tissue-supporting implant or filler in a body.

9. The medical grade nanofibrillar cellulose hydrogel of 6 or 7 for use as a dermal filler in a body.

10. The medical grade nanofibrillar cellulose hydrogel of claim 6 or 7 for use as viscoelastic supplementation for treatment of arthritis or other joint conditions.

11. The medical grade nanofibrillar cellulose hydrogel of claim 6 or 7 for use as a barrier agent in surgery.

12. A method for manufacturing medical grade nanofibrillar cellulose hydrogel, the method comprising
- providing a suspension of chemically and/or enzymatically unmodified plant-based, such as wood-based, cellulose pulp,
- pre-refining the cellulose pulp suspension with a refiner to obtain a pre-refined cellulose having SR value 87 or more, such as SR value in the range of 87-95,
- dispersing the pre-refined cellulose pulp to obtain a concentration of pre-refined cellulose in the range 1.6-3.6% by weight, such as 1.65-3.20% by weight,
- disintegrating the pre-refined cellulose pulp by a disintegrating device at said concentration to obtain medical grade nanofibrillar cellulose hydrogel,
- recovering the medical grade nanofibrillar cellulose hydrogel from the disintegrating device without adjusting the concentration,
- to obtain nanofibrillar cellulose hydrogel having a concentration of nanofibrillar cellulose in the range of 1.4-3.4% by weight, such as 1.45-3.0% by weight.

13. The method of claim 12, wherein the disintegrating device comprises a microfludizer utilizing one or more interaction chambers, such as wherein the disintegrating of the pre-refined cellulose pulp comprises
- providing a microfluidizer comprising a first interaction chamber and a second interaction chamber as the disintegration device,
- carrying out a first disintegration run in a first interaction chamber, and
- carrying out one or more subsequent disintegration runs in a second interaction chamber having a smaller channel diameter compared to the first chamber.

14. The method of claim 12 or 13, wherein the refiner is a mill with a roll with vertical cutting bars in a container, such as a PFI type of refiner mill.

15. The method of any of the claims 12-14, wherein the cellulose comprises birch cellulose, preferably wherein the cellulose consists of birch cellulose.

16. The method of any of the claims 12-15, wherein the pre-refined cellulose pulp is disintegrated until the obtained nanofibrillar cellulose provides a storage modulus in the range of 1-40 Pa determined by a rotational rheometer using plate geometry at a consistency of 0.5% by weight in aqueous medium at 22±1°C and/or a storage modulus in the range of 0.7-20 Pa determined by a rotational rheometer using vane geometry at a consistency of 0.5% by weight in aqueous medium at 22±1°C, and/or until the obtained nanofibrillar cellulose has a number-average diameter of fibrils and/or fibril bundles of 100 nm or less, preferably until the obtained nanofibrillar cellulose provides a zero-shear viscosity in the range of 1500-50000 Pa·s and a yield stress in the range of 0.5-10 Pa, determined by a rotational rheometer using plate geometry at a consistency of 0.5% by weight in aqueous medium at 22±1°C.

17. The method of any of the claims 12-16, wherein the method is carried out in the absence of active agents, and preferably the obtained and provided medical grade nanofibrillar cellulose hydrogel comprises no active agents.

18. The medical grade nanofibrillar cellulose hydrogel of any of the claims 1 -9 obtained with the method of any of the claims 12-17.
